**Europäisches Patentamt**

**European Patent Office**

**Office européen des brevets**

(11) Veröffentlichungsnummer : **0 455 081 B1**

(12) **EUROPÄISCHE PATENTSCHRIFT**

(45) Veröffentlichungstag der Patentschrift :
**29.06.94 Patentblatt 94/26**

(21) Anmeldenummer : **91106394.9**

(22) Anmeldetag : **20.04.91**

(51) Int. Cl.⁵ : **A61K 7/06,** A61K 7/11,
C08F 26/06

(54) **Haarfestigungs- und Haarpflegemittel.**

(30) Priorität : **30.04.90 DE 4013872**

(43) Veröffentlichungstag der Anmeldung :
**06.11.91 Patentblatt 91/45**

(45) Bekanntmachung des Hinweises auf die
Patenterteilung :
**29.06.94 Patentblatt 94/26**

(84) Benannte Vertragsstaaten :
**DE ES FR GB IT NL**

(56) Entgegenhaltungen :
**EP-A- 0 074 191**
**EP-A- 0 088 964**
**US-A- 3 145 147**

(56) Entgegenhaltungen :
**SOFW: SEIFEN, OLE, FETTE, WACHSE, Band
116, Nr. 4, 1. März 1990, Seiten 130-137, Augsburg, DE; H. -U. WEKEL et al.: "Moderne Haar-
sprayformulierungen/Trends und neue
Polymere"**

(73) Patentinhaber : **BASF Aktiengesellschaft
Carl-Bosch-Strasse 38
D-67063 Ludwigshafen (DE)**

(72) Erfinder : **Potthoff-Karl, Birgit, Dr.
Grundelbachstrasse 112 e
W-6940 Weinheim (DE)**
Erfinder : **Sperling-Vietmeier, Karin, Dr.
Im Kirchestueck 12
W-6730 Neustadt (DE)**
Erfinder : **Sanner, Axel, Dr.
Lorscher Ring 2 c
W-6710 Frankenthal (DE)**

EP 0 455 081 B1

**Beschreibung**

Die vorliegende Erfindung betrifft Haarfestigungs- und Haarpflegemittel, welche neben den hierfür üblichen Bestandteilen als Filmbildner Polymerisate auf der Basis von N-Vinylcaprolactam enthalten, die aus Gruppe I

- 35 bis 90 Gew.-% N-Vinylcaprolactam (Monomeres A)
- 10 bis 65 Gew.-% N-Vinylimidazol (Monomeres B)
- 0 bis 4 Gew.-% weiterer radikalisch copolymerisierbarer Monomerer E

oder Gruppe II

- 35 bis 65 Gew.-% N-Vinylcaprolactam (Monomeres A)
- 35 bis 65 Gew.-% einer Mischung aus 5 bis 50 Gew.-Teilen N-Vinylimidazol (Monomeres 3) und 10 bis 60 Gew.-Teilen N-Vinylpyrrolidon (Monomeres C)
- 0 bis 4 Gew.% weiterer radikalisch copolymerisierbarer Monomerer E

oder Gruppe III

- 35 bis 85 Gew.-% N-Vinylcaprolactam (Monomeres A)
- 15 bis 65 Gew.-% einer Mischung aus 5 bis 15 Gew.-Teilen N-Vinylimidazol (Monomeres B) und 10 bis 35 Gew.-Teilen eines Alkylacrylates oder Alkylmethacrylates mit 1 bis 4 C-Atomen im Alkylrest (Monomeres D)
- 0 bis 4 Gew.-% weiterer radikalisch copolymerisierbarer Monomerer E

oder Gruppe IV

- 35 bis 75 Gew.-% N-Vinylcaprolactam (Monomeres A)
- 25 bis 65 Gew.-% einer Mischung aus 20 bis 60 Gew.-Teilen des Monomeren D und 5 bis 15 Gew.-Teilen Acrylsäure der Methacrylsäure (Monomeres F)
- 0 bis 4 Gew.-% weiterer radikalisch copolymerisierbarer Monomerer E

oder Gruppe V

- 35 bis 80 Gew.-% N-Vinylcaprolactam (Monomeres A)
- 20 bis 65 Gew.-% einer Mischung aus 15 bis 50 Gew.-Teilen des Monomeren D und 5 bis 15 Gew.-Teilen eines Alkylacrylates oder Alkylmethacrylates mit 1 bis 4 C-Atomen im Alkylrest, wobei dieser zusätzlich eine unsubstituierte oder mit $C_1$-$C_4$-Alkylresten substituierte Aminogruppe trägt (Monomeres G)
- 0 bis 4 Gew.-% weiterer radikalisch copolymerisierbarer Monomerer E

aufgebaut sind, wobei vorhandene Carboxylgruppen der Polymerisate teilweise oder vollständig durch Amine neutralisiert sind.

Ein Teil der Polymerisate sind neue Stoffe. Deshalb betrifft die Erfindung weiterhin diese neuen Stoffe.

Die DE-A 32 27 334 (1) betrifft Copolymerisate aus 20 bis 75 Gew.-Teilen eines $C_2$- bis $C_{20}$-Alkylester der Acrylsäure oder Methacrylsäure, 5 bis 50 Gew.-Teilen eines stickstoffhaltigen, neutral reagierenden, wasserlöslichen Monomeren, beispielsweise N-Vinylcaprolactam oder N-Vinylpyrrolidon, 1 bis 25 Gew.-Teilen eines kationische Gruppen enthaltenden Monomeren, beispielsweise N-Vinylimidazol, und 1 bis 25 Gew.-Teilen einer copolymerisierbaren, olefinisch ungesättigten $C_3$- bis $C_5$-Carbonsäure, beispielsweise Acrylsäure oder Methacrylsäure. Die Copolymerisate dienen als Filmbildner in Haarbehandlungsmitteln.

Die EP-B 074 191 (2) betrifft eine Haarkonditionierungszusammensetzung, welche ein Terpolymer aus einem größeren Anteil N-Vinylcaprolactam, einem kleineren Anteil N-Vinylpyrrolidon und einem Ammoniumderivatmonomer, z.B. einem Dialkylaminoalkylacrylat, enthält.

Die US-A 3 145 147 (3) betrifft eine filmbildende Zusammensetzung, welche ein Copolymer aus 80 bis 95 Gew.-% N-Vinylcaprolactam und 5 bis 20 Gew.-% eines polymerisierbaren Monomeren, nämlich eines Vinylesters mit 3 bis 6 C-Atomen, eines Alkylacrylates mit 4 bis 5 C-Atomen oder eines Acrylamides, Acrylonitrils oder Alkylvinylethers mit jeweils 3 bis 4 C-Atomen, umfaßt.

In der DE-C 12 61 822 (4) werden Mischpolymerisate von N-Vinylcaprolactam mit beispielsweise N-Vinylimidazol oder mit N-Vinylimidazol und N-Vinylpyrrolidon beschrieben. Die Mischpolymerisate dienen als Mittel zur Verminderung der Pigmentwanderung beim Färben von Fasermaterial mit Pigmentfarbstoff-Flotten.

Die DE-A 21 12 549 (5) betrifft wasserlösliche Tetrapolymere aus einem N-Vinyllactam, beispielsweise N-Vinylpyrrolidon oder N-Vinylcaprolactam, einem Alkylacrylester, einem Alkylmethacrylester und einer olefinisch ungesättigten Carbonsäure. Das N-Vinyllactam wird vorzugsweise in einer Menge von 10 bis 30 Gew.-%, bezogen auf das Tetrapolymer, eingesetzt. Die Tetrapolymere dienen als Filmbildner für Überzüge, Textilschlichten, Klebstoffe, Haarspray, Bindemittel für Sandkörner und zur Herstellung von Raketenhülsen.

Für die Haarkosmetik werden in zunehmendem Maße Sprayzubereitungen mit Kohlenwasserstoffen anstelle von halogenierten Kohlenwasserstoffen als Treibmittel eingesetzt. Die hierbei als Filmbildner verwendeten, zum oben angeführten Stand der Technik gehörigen Copolymerisate zeigen noch teilweise verbesserungsbedürftige Werte für die Verträglichkeit mit den unpolaren Kohlenwasserstoffen der Sprayzubereitungen, d.h. die Löslichkeit in ihnen ist noch nicht hoch genug. Außerdem läßt meist die haarfestigende Wirkung dieser Copolymerisate noch zu wünschen übrig. Weiterhin ist oft noch die Wasseraufnahmebereitschaft der mit diesen Copolymerisaten behandelten Haare zu hoch.

Aufgabe der vorliegenden Erfindung war es, Filmbildner für die Haarkosmetik bereitzustellen, die sich durch eine gute Verträglichkeit mit unpolaren Treibmitteln auf der Basis von Kohlenwasserstoffen auszeichnen und gleichzeitig eine gute haarfestigende Wirkung und geringe Wasseraufnahme zeigen.

Demgemäß wurden die eingangs definierten Haarfestigungs- und Haarpflegemittel gefunden.

Unter N-Vinylcaprolactam (Monomeres A) ist N-vinyl-ε-caprolactam zu verstehen.

Als Monomere D sind vor allem die Methyl-, Ethyl-, n-Propyl-, iso-Propyl, n-Butyl-, iso-Butyl-, sek.-Butyl- und tert.-Butylester der Acrylsäure und Methacrylsäure zu nennen. Bevorzugt werden hiervon die Butylester und vor allem die tert.-Butylester.

Zur geringfügigen Abänderung der Eigenschaften des Polymerisates können weitere radikalisch copolymerisierbare Monomere E in einer Menge bis zu 4 Gew.-%, insbesondere bis zu 3 Gew.-%, mitenthalten sein. Als Monomere E dienen z.B. Vinylacetat, Vinylpropionat oder Hydroxypropylacetat.

Als Monomer F werden Acrylsäure und vorzugsweise Methacrylsäure eingesetzt.

Als Monomere G können vor allem Methyl-, Ethyl-, n-Propyl- und n-Butyl-ester der Acrylsäure und Methacrylsäure verwendet werden, welche insbesondere am Ende dieser Alkylgruppe eine unsubstituierte oder vorzugsweise mono- oder disubstituierte Aminogruppe tragen. Besonders bevorzugt werden 2-(Dialkylamino)ethylmethacrylate wie 2-(Dimethylamino)- und 2-(Diethylamino)ethylmethacrylat.

In den Polymerisaten enthaltene Carboxylgruppen werden mit einem Amin teilweise oder vollständig, zweckmäßigerweise zu 5 bis 100 %, vorzugsweise zu 30 bis 90 %, neutralisiert. Die Neutralisation erfolgt bevorzugt mit

- einem Mono-, Di- oder Trialkanolamin mit 2 bis 5 C-Atomen im Alkanolrest, der gegebenenfalls in veretherter Form vorliegt, beispielsweise Mono-, Di- und Triethanolamin, Mono-, Di- und Tri-n-propanolamin, Mono-, Di- und Triisopropanolamin, 2-Amino-2-methylpropanol und Di(2-methoxyethyl)amin,
- einem Alkandiolamin mit 2 bis 5 C-Atomen, beispielsweise 2-Amino-2-methylpropan-1,3-diol und 2-Amino-2-ethylpropan-1,3-diol, oder
- einem primären, sekundären oder tertiären Alkylamin mit insgesamt 5 bis 10 C-Atomen, beispielsweise N,N-Diethylpropylamin.

Besonders gute Ergebnisse bei der Neutralisation erzielt man mit 2-Amino-2-methylpropanol, Triisopropanolamin und 2-Amino-2-ethylpropan-1,3-diol.

Die Monomerkombinationen A + B + C und insbesondere A + B + D, A + D + F sowie A + D + G, gegebenenfalls jeweils mit geringen Mengen an E, stellen besonders bevorzugte Ausführungsformen dar.

Die Polymerisate werden durch radikalische Polymerisation oder Copolymerisation der Monomeren A bis G hergestellt. Hierbei arbeitet man nach den üblichen Polymerisationstechniken, zum Beispiel nach den Methoden der Suspensions-, Emulsions- oder Lösungspolymerisation.

Als besonders zweckmäßig hat sich die Lösungspolymerisation in einem organischen Lösungsmittel, in der Regel einem Alkohol, herausgestellt. Man arbeitet hier üblicherweise bei Temperaturen von 60 bis 130°C, wobei die Umsetzung bei Normaldruck oder unter Eigendruck durchgeführt werden kann.

Als Initiatoren für die radikalisch ablaufende Polymerisationsreaktion werden die üblichen Peroxo- oder Azoverbindungen, beispielsweise Dibenzoylperoxid, tert.-Butylperpivalat, tert.-Butylper-2-ethylhexanoat, Di-tert.-butylperoxid, tert.-Butylhydroperoxid, 2,5-Dimethyl-2,5-di-(tert.-butyl-peroxy)hexan oder Azo-bis-isobutyronitril, zweckmäßigerweise in Mengen von 0,1 bis 2 Gew.-%, bezogen auf das Gewicht der Monomeren, eingesetzt.

Man wählt die Mengen an Monomeren und Lösungsmittel zweckmäßigerweise so, daß man 30 bis 80 gew.-%ige Lösungen der Copolymerisate erhält. Dabei kann vorliegendes organisches Lösungsmittel nach den üblichen Methoden, beispielsweise durch Destillation, entfernt und durch Wasser ersetzt werden, falls ein Haarfestigungs- oder Haarpflegemittel auf wäßriger Basis gewünscht wird.

Die Polymerisate sollen K-Werte von 10 bis 60, vorzugsweise 20 bis 55, aufweisen. Der jeweils gewünschte K-Wert läßt sich in an sich bekannter Weise durch Wahl der Polymerisationsbedingungen, beispielsweise der Polymerisationsdauer und der Initiatorkonzentration, einstellen. Die K-Werte werden nach Fikentscher, Cellulosechemie, Bd. 13, S. 58 bis 64 (1932) bei 25°C in 1 gew.-%iger ethanolischer Lösung gemessen und stellen ein Maß für das Molgewicht dar.

Derartige Polymerisate haben üblicherweise Glasübergangstemperaturen zwischen 80 und 180, insbesondere zwischen 90 und 150°C.

Ein Teil der Polymerisate sind neue Stoffe. Deshalb sind auch Gegenstand der vorliegenden Erfindung Copolymerisate, welche durch radikalische Polymerisation von

- 35 bis 95 Gew.-% N-Vinylcaprolactam (Monomeres A)

- 5 bis 65 Gew.-% einer Mischung aus 5 bis 15 Gew.-Teilen N-Vinylimidazol (Monomeres B) und 10 bis 35 Gew.-Teilen des Monomeren D
- 0 bis 4 Gew.-% weiterer radikalisch copolymerisierbarer Monomerer E

erhältlich sind. Diese Polymerisate haben meist K-Werte zwischen 10 und 60, insbesondere zwischen 20 und 55.

Weiterhin sind Gegenstand der vorliegenden Erfindung Copolymerisate, welche durch radikalische Polymerisation von

- 35 bis 95 Gew.-% N-Vinylcaprolactam (Monomeres A)
- 5 bis 65 Gew.-% einer Mischung aus 15 bis 50 Gew.-Teilen des Monomeren D und 5 bis 15 Gew.-Teilen des Monomeren G
- 0 bis 4 Gew.-% weiterer radikalisch copolymerisierbarer Monomerer E

erhältlich sind. Diese Polymerisate haben meist K-Werte zwischen 10 und 60, insbesondere zwischen 20 und 55.

Die erfindungsgemäßen Haarfestigungs- und Haarpflegemittel kommen beispielsweise als Haarfestigerlösungen, Haarschäume, Haargele und vor allem als Frisurenfestiger in Form von Sprayzubereitungen zur Anwendung.

Eine zweckmäßige Zubereitung für Haarfestigerlösungen enthält:

- 1 bis 20 Gew.-% des gegebenenfalls teilweise oder vollständig neutralisierten Polymerisates;
- 0 bis 99 Gew.-% eines üblichen Lösungsmittels wie vor allem Aceton, Ethanol, n-Propanol, Isopropanol und 1-Methoxypropan-2-ol oder deren Gemische;
- 0 bis 99 Gew.-% Wasser.

Eine bevorzugte Haarfestigerlösung ist überwiegend wäßrig und enthält 2 bis 15 Gew.-% Polymerisat, 60 bis 98 Gew.-% Wasser und gegebenenfalls als Rest zu 100 Gew.-% eines der oben genannten Lösungsmittel oder deren Gemische.

Eine zweckmäßige und vorteilhafte Zusammensetzung für Haarschäume ergibt sich nach folgender Vorschrift:

- 1 bis 15 Gew.-%, vorzugsweise 2 bis 10 Gew.-% des gegebenenfalls teilweise oder vollständig neutralisierten Polymerisates;
- 5 bis 90 Gew.-%, vorzugsweise 60 bis 85 Gew.-% Wasser;
- 0 bis 20 Gew.-% eines üblichen Lösungsmittels wie vor allem Aceton, Ethanol, n-Propanol, Isopropanol und 1-Methoxypropan-2-ol oder deren Gemische;
- 10 bis 50 Gew.-% eines üblichen Treibmittels wie Propan, n-Butan, Isobutan, 2,2-Dimethylpropan, Isopentan und Dimethylether und deren Gemische.

Diesen Zusammensetzungen werden, bezogen auf das Gesamtgewicht, etwa 0,1 bis etwa 1 Gew.-% dem Fachmann bekannte Hilfsmittel zur Schaumbildung und Schaumstabilisierung zugesetzt.

Selbstverständlich kommen für die oben genannten Haarfestiger- und Haarschaum-Zubereitungsformen auch weitere übliche Zusätze, wie Parfüm, Konservierungsstoffe, u.a., in den hierfür üblichen Mengen in Betracht.

Bei den Haarsprayzubereitungen werden besonders solche bevorzugt, die die folgenden Bestandteile enthalten:

- 0,1 bis 20, vorzugsweise 0,5 bis 12, insbesondere 2 bis 10 Gew.-% des gegebenenfalls teilweise oder vollständig neutralisierten Polymerisates;
- 10 bis 95, vorzugsweise 20 bis 60, insbesondere 25 bis 50 Gew.-% eines üblichen Lösungsmittels wie vor allem Ethanol und Isopropanol und daneben auch Aceton, n-Propanol, n-Butanol, 2-Methoxypropan-1-ol, n-Pentan, n-Hexan, Cyclohexan, n-Heptan oder Dichlormethan oder deren Gemische;
- 5 bis 90, vorzugsweise 30 bis 80, insbesondere 45 bis 70 Gew.-% eines üblichen Treibmittels wie Propan, n-Butan, Isobutan, 2,2-Dimethylbutan, Isopentan, Dimethylether, Fluortrichlormethan, Dichlordifluormethan oder Dichlortetrafluorethan oder deren Gemische. Als Treibmittel (Treibgase) kommen von den genannten Verbindungen vor allem die Kohlenwasserstoffe und insbesondere Propan und n-Butan - ein Gemisch im Gewichtsverhältnis von beispielsweise 40 : 60 oder 25 : 75 oder n-Butan alleine - zur Anwendung. Gegebenenfalls werden einer oder mehrere der genannten Fluorchlorkohlenwasserstoffe in Treibmittelmischungen mitverwendet, jedoch nur in geringen Mengen, etwa bis zu 20 Gew.-%, bezogen auf die Treibmittelmischung.

Außerdem können diese Sprayzubereitungen noch geringe Mengen an Parfümölen, beispielsweise 0,1 bis 5,0 Gew.-%, enthalten.

Die üblichen Bestandteile und Zusammensetzungen anderer Haarfestigungs- und Haarpflegemittel sind dem Fachmann bekannt und brauchen deshalb hier nicht näher erläutert zu werden.

Die in den erfindungsgemäßen Haarfestigungs- und Haarpflegemitteln enthaltenen Polymerisate zeichnen sich durch ihre hohe Verträglichkeit mit den unpolaren Treibmitteln in Sprayzubereitungen, insbesondere mit Kohlenwasserstoffen wie Propan oder n-Butan oder ihrem Gemisch aus. In der Regel erreicht man mit ihnen Verträglichkeitswerte zwischen 50 und 85, insbesondere zwischen 70 und 85 Gew.-% bei einer gleichzeitig außergewöhnlich guten haarfestigenden Wirkung, ersichtlich an den hohen Werten für die Curl-Retention, die hierbei normalerweise zwischen 70 und 95, insbesondere zwischen 85 und 95 % liegen. Die Wasseraufnahmebereitschaft der behandelten Haare ist gering, sie liegt bei 3 bis 18, insbesondere 3 bis 10 Gew.-%, wodurch die Frisur einen längeren Halt bekommt und weniger klebt.

Weiterhin zeichnen sich die erfindungsgemäßen Haarfestigungs- und Haarpflegemittel dadurch aus, daß sie das Haar praktisch nicht verkleben und es gut auskämmbar bleibt. Die behandelten Haare zeigen ein natürliches Aussehen. Der mit erfindungsgemäßen Mitteln erzielte Steifeffekt der Haare ist durchweg gut.

Beispiele

Die Herstellung der an sich bekannten, in den erfindungsgemäßen Haarfestigungs- und Haarpflegemitteln eingesetzten Polymerisate erfolgte nach den üblichen Methoden der Lösungspolymerisation. Stellvertretend für alle übrigen Herstellungsvorschriften sei nachfolgend die Synthese des Polymerisates aus Beispiel 10 beschrieben.

Herstellung eines Polymerisates aus 60 Gew.-% N-Vinylcaprolactam, 10 Gew.-% N-Vinylimidazol und 30 Gew.-% tert.-Butylacrylat

Eine Lösung von 30 g N-Vinylcaprolactam, 5 g N-Vinylimidazol, 15 g tert.-Butylacrylat und 0,53 g tert.-Butylperpivalat (75 gew.-%ig) in 275 g Ethanol wurde auf 75°C erwärmt. Nach dem Anspringen der Polymerisation, erkennbar an einer Viskositätserhöhung, wurden gleichzeitig eine Mischung aus 270 g N-Vinylcaprolactam, 45 g N-Vinylimidazol, 135 g tert.-Butylacrylat und 100 g Ethanol und eine Lösung von 2,4 g tert.-Butylperpivalat (75 gew.-%ig) in 60 g Ethanol im Laufe von 3 Stunden zugegeben, wobei die Temperatur bei schwachem Sieden auf 77 bis 80°C gehalten wurde. Anschließend wurde bei der gleichen Temperatur eine Lösung von 2,4 g tert.-Butylperpivalat (75 gew.-%ig) in 60 g Ethanol innerhalb von weiteren 3 Stunden zugetropft.

Danach wurden 3,0 g 2,5-Dimethyl-2,5-di-(tert.-butyl-peroxy)hexan auf einmal zugegeben. Das Reaktionsgefäß wurde druckfest verschlossen, auf 130°C erwärmt und 3 Stunden bei dieser Temperatur gehalten.

Eigenschaften der Polymerisate

Die folgenden Tabellen 1 bis 6 zeigen die Daten für Zusammensetzung, K-Wert, Kohlenwasserstoffverträglichkeit, haarfestigende Wirkung und Wasseraufnahme der in den erfindungsgemäßen Mitteln eingesetzten Polymerisate.

Der K-Wert wurde in 1,0 gew.-%iger ethanolischer Lösung bei 25°C gemessen.

Der Wert für die Kohlenwasserstoffverträglichkeit mit einer Propan/n-Butan-Mischung im Gewichtsverhältnis von 25 : 75 gibt an, wieviel Gew.-% dieses Treibgasgemisches eine Sprayzubereitung, die neben Ethanol als Lösungsmittel 3 Gew.-% des bei Vorliegen von Carboxylgruppen zu 75 % mit 2-Amino-2-methylpropanol neutralisierten Copolymeren aufweist, maximal enthalten darf, ohne daß bei 0°C eine Trübung auftritt.

Die Curl-Retention ist ein Maß für die haarfestigende Wirkung. Sie wird im Modellversuch an Haarlocken gemessen, die durch eine übliche Wasserwelle an ca. 15 cm langen Haaren erzeugt und mit der jeweiligen Sprayzubereitung aus 10 cm Entfernung 4 sec lang besprüht worden sind. Nach einer Verweilzeit von 5 Stunden der aufgehängten Locken in einer Klimakammer bei 25°C und 90 % relativer Luftfeuchtigkeit wird die relative Verformung (Aufweitung) der Lockert, bezogen auf ihre ursprüngliche Form, bestimmt. Ein hoher Wert bedeutet eine hohe Festigkeitswirkung, d.h. bei 100 % bliebe die ursprüngliche Form vollständig erhalten.

Die Curl-Retention für die in den Tabellen 1 bis 6 bezeichneten Polymerisate wurde jeweils mit der folgenden Standard-Sprayformulierung bestimmt:

6,3 Gew.-%      Polymerisat, welches bei Vorliegen von Carboxylgruppen zu 75 % mit 2-Amino-2-methylpropanol neutralisiert worden war,

33,7 Gew.-%      Ethanol und

60,0 Gew.-%      Propan/n-Butan (25 : 75)

Die Wasseraufnahme der mit den Polymerisaten behandelten Haare aus der umgebenden Luft wurde nach 7 Tagen bei 75 % relativer Luftfeuchtigkeit gemessen.

Tabelle 1

Polymerisate aus den Monomeren A und E   (zum Vergleich)

| Bsp. | Zusammensetzung [Gew.-%] | K-Wert | Kohlenwasser- stoffverträg- lichkeit mit Propan/n-Butan (25:75) [Gew.-%] | Curl- Reten- tion [%] | Wasser- aufnahme [Gew.-%] |
|---|---|---|---|---|---|
| 1 | 100 N-Vinylcaprolactam | 32,1 | 75 | 82 | 9,3 |
| 2 | 97 N-Vinylcaprolactam 3 Vinylacetat | 20,4 | 74 | 83 | 9,4 |
| A | 80 N-Vinylcaprolactam 20 Vinylacetat | 29,8 | 72 | 83 | 9,7 |

Beispiel A wurde gemäp Literaturstelle (3) hergestellt; die wäprige Lösung von A war im Gegensatz zu denen von Beispiel 1 und 2 gefärbt und die mit A behandelten Haare waren grau und schuppig.

Tabelle 2

Polymerisate aus den Monomeren A und B

| Bsp. | Zusammensetzung | K-Wert | Kohlenwasser-stoffverträg-lichkeit mit Propan/n-Butan | Curl-Reten-tion | Wasser-aufnahme |
|------|-----------------|--------|------|------|------|
| | [Gew.-%] | | (25:75) [Gew.-%] | [%] | [Gew.-%] |
| 3 | 40 N-Vinylcaprolactam 60 N-Vinylimidazol | 45,0 | 50 | 83 | 18,0 |
| 4 | 50 N-Vinylcaprolactam 50 N-Vinylimidazol | 43,9 | 56 | 85 | 14,7 |
| 5 | 60 N-Vinylcaprolactam 40 N-Vinylimidazol | 42,8 | 57 | 87 | 16,6 |
| 6 | 70 N-Vinylcaprolactam 30 N-Vinylimidazol | 43,2 | 61 | 88 | 15,4 |
| 7 | 80 N-Vinylcaprolactam 20 N-Vinylimidazol | 43,6 | 65 | 91 | 13,7 |

Tabelle 3

Polymerisate aus den Monomeren A, B und C

| Bsp. | Zusammensetzung [Gew.-%] | K-Wert | Kohlenwasser-stoffverträg-lichkeit mit Propan/n-Butan (25:75) [Gew.-%] | Curl-Reten-tion [%] | Wasser-aufnahme [Gew.-%] |
|---|---|---|---|---|---|
| 8 | 35 N-Vinylcaprolactam<br>50 N-Vinylimidazol<br>15 N-Vinylpyrrolidon | 46,1 | 52 | 79 | 18,0 |
| 9 | 60 N-Vinylcaprolactam<br>30 N-Vinylimidazol<br>10 N-Vinylpyrrolidon | 40,4 | 63 | 93 | 14,1 |

Tabelle 4

Polymerisate aus den Monomeren A, B und D

| Bsp. | Zusammensetzung [Gew.-%] | K-Wert | Kohlenwasser-stoffverträg-lichkeit mit Propan/n-Butan (25:75) [Gew.-%] | Curl-Reten-tion [%] | Wasser-aufnahme [Gew.-%] |
|---|---|---|---|---|---|
| 10 | 60 N-Vinylcaprolactam 10 N-Vinylimidazol 30 tert.-Butylacrylat | 27,6 | 76 | 89 | 5,8 |
| 11 | 75 N-Vinylcaprolactam 5 N-Vinylimidazol 20 tert.-Butylacrylat | 29,3 | 75 | 87 | 6,4 |
| 12 | 75 N-Vinylcaprolactam 5 N-Vinylimidazol 20 Methylacrylat | 27,8 | 60 | 86 | 8,0 |
| 13 | 75 N-Vinylcaprolactam 5 N-Vinylimidazol 20 Ethylacrylat | 53,0 | 74 | 84 | 7,1 |

Tabelle 5

Polymerisate aus den Monomeren A, D und F

| Bsp. | Zusammensetzung [Gew.-%] | K-Wert | Kohlenwasser-stoffverträg-lichkeit mit Propan/n-Butan (25:75) [Gew.-%] | Curl-Reten-tion [%] | Wasser-aufnahme [Gew.-%] |
|---|---|---|---|---|---|
| 14 | 40 N-Vinylcaprolactam 55 tert.-Butylacrylat 5 Methacrylsäure | 24,2 | 77 | 90 | 4,0 |
| 15 | 40 N-Vinylcaprolactam 50 tert.-Butylacrylat 10 Methacrylsäure | 27,6 | 71 | 90 | 5,7 |
| 16 | 40 N-Vinylcaprolactam 45 tert.-Butylacrylat 15 Methylacrylsäure | 26,3 | 61 | 89 | 6,5 |
| 17 | 50 N-Vinylcaprolactam 45 tert.-Butylacrylat 5 Methacrylsäure | 25,1 | 75 | 90 | 5,4 |
| 18 | 60 N-Vinylcaprolactam 35 tert.-Butylacrylat 5 Methacrylsäure | 26,9 | 75 | 90 | 7,1 |
| 19 | 70 N-Vinylcaprolactam 25 tert.-Butylacrylat 5 Methacrylsäure | 28,7 | 75 | 88 | 7,3 |
| 20 | 70 N-Vinylcaprolactam 25 n-Butylacrylat 5 Methacrylsäure | 29,4 | 74 | 82 | 9,3 |
| 21 | 70 N-Vinylcaprolactam 20 n-Butylacrylat 10 Methacrylsäure | 29,3 | 66 | 70 | 10,0 |

Tabelle 5 (Fortsetzung)

| Bsp. | Zusammensetzung [Gew.-%] | K-Wert | Kohlenwasser-stoffverträg-lichkeit mit Propan/n-Butan (25:75) [Gew.-%] | Curl-Reten-tion [%] | Wasser-aufnahme [Gew.-%] |
|---|---|---|---|---|---|
| 22 | 75 N-Vinylcaprolactam<br>20 n-Butylmethacrylat<br>5 Methacrylsäure | 33,3 | 75 | 84 | 6,3 |
| 23 | 70 N-Vinylcaprolactam<br>25 Ethylacrylat<br>5 Methacrylsäure | 27,6 | 64 | 72 | 7,4 |
| Zum Vergleich: | | | | | |
| B | 20 N-Vinylpyrrolidon<br>9 N-Vinylimidazol<br>64 tert.-Butylacrylat<br>7 Acrylsäure | 17,0 | 65 | 22 | 7,4 |

Beispiel B entspricht dem Beispiel 5 aus Literaturstelle (1).

Tabelle 6

Polymerisate aus den Monomeren A, D und G

| Bsp. | Zusammensetzung [Gew.-%] | K-Wert | Kohlenwasser- stoffverträg- lichkeit mit Propan/n-Butan (25:75) [Gew.-%] | Curl- Reten- tion [%] | Wasser- aufnahme [Gew.-%] |
|---|---|---|---|---|---|
| 24 | 45 N-Vinylcaprolactam<br>45 tert.-Butylacrylat<br>10 DMAEMA | 24,3 | 80 | 95 | 3,3 |
| 25 | 55 N-Vinylcaprolactam<br>40 tert.-Butylacrylat<br>5 DMAEMA | 24,4 | 80 | 96 | 3,8 |
| 26 | 60 N-Vinylcaprolactam<br>35 tert.-Butylacrylat<br>5 DMAEMA | 25,6 | 75 | 90 | 7,1 |
| 27 | 65 N-Vinylcaprolactam<br>30 tert.-Butylacrylat<br>5 DMAEMA | 27,3 | 78 | 96 | 5,3 |
| 28 | 75 N-Vinylcaprolactam<br>20 tert.-Butylacrylat<br>5 DMAEMA | 27,2 | 76 | 86 | 6,9 |
| 29 | 65 N-Vinylcaprolactam<br>30 n-Butylmethacrylat<br>5 DMAEMA | 39,8 | 75 | 93 | 6,3 |
| 30 | 75 N-Vinylcaprolactam<br>20 n-Butylmethacrylat<br>5 DMAEMA | 33,3 | 84 | 75 | 6,7 |

Tabelle 6 (Fortsetzung)

| Bsp. Zusammensetzung | K-Wert | Kohlenwasser-stoffverträg-lichkeit mit Propan/n-Butan | Curl-Reten-tion | Wasser-aufnahme |
|---|---|---|---|---|
| [Gew.-%] | | (25:75) [Gew.-%] | [%] | [Gew.-%] |

Zum Vergleich:

| C 47,5 N-Vinylcaprolactam 47,5 N-Vinylpyrrolidon 5 DMAEMA | 43,9 | 80 | 70 | 3,9 |

DMAEMA: 2-(Dimethylamino)ethylmethacrylat

Beispiel C entspricht dem dritten Beispiel aus Tabelle III der Literaturstelle (2); dort wurde die Curl-Retention mit 89 bis 90 %, allerdings bei wesentlich schonenderen Bedingungen (45 Minuten statt 5 Stunden), bestimmt.

Formulierungsbeispiele für Haarfestigungs- und Haarpflegemittel

Beispiel 31 (zum Vergleich)

Haarfestigerlösung mit Alkohol

10,0 Gew.-%     N-Vinylcaprolactam-Polymerisat aus Beispiel 1
5,0 Gew.-%     Ethanol
85,0 Gew.-%     Wasser
     Die erhaltene Lösung war schwach opal.

Beispiel 32

Haarfestigerlösung ohne Alkohol

10,3 Gew.-%     N-Vinylcaprolactam/tert.-Butylacrylat/Methacrylsäure-Polymerisat aus Beispiel 19, welches in zu 100 % mit 2-Amino-2-methylpropanol neutralisierter Form vorliegt
0,2 Gew.-%     übliches Konservierungsmittel
89,5 Gew.-%     Wasser

Beispiel 33

Haarschaum mit Festiger

6,0 Gew.-%     N-Vinylcaprolactam-Polymerisat aus Beispiel 9
0,1 Gew.-%     mit 25 mol Ethylenoxid umgesetzter Cetyl-Stearyl-Alkohol
0,3 Gew.-%     Cetyl-dimethyl-2-hydroxyethyl-ammoniumdihydrogenphosphat
0,4 Gew.-%     übliches Parfüm
0,2 Gew.-%     übliches Konservierungsmittel
83,0 Gew.-%     Wasser

10,0 Gew.-%     Propan/n-Butan (25:75)

Man erhielt schönen, festen und etwas cremigen Schaum.

Beispiel 34

Haarschaum mit Conditioner und Festiger

4,0 Gew.-%     N-Vinylcaprolactam-Polymerisat aus Beispiel 9
5,0 Gew.-%     Vinylpyrrolidon-Vinylimidazoliummethochlorid-Copolymerisat
0,5 Gew.-%     Cetyl-dimethyl-2-hydroxyethyl-ammoniumdihydrogenphosphat
0,4 Gew.-%     übliches Parfüm
0,2 Gew.-%     übliches Konservierungsmittel
79,9 Gew.-%     Wasser
10,0 Gew.-%     Propan/n-Butan (25:75)

Man erhielt einen festen, trockenen Schaum.

Beispiel 35

Haargel

6,0 Gew.-%     -Vinylcaprolactam-Polymerisat aus Beispiel 9
0,6 Gew.-%     Polyacrylsäure
11,0 Gew.-%     N,N,N',N'-Tetrakis-(2-hydroxypropyl)-ethylendiamin
20,0 Gew.-%     Ethanol
62,4 Gew.-%     Wasser

Man erhielt ein opakes Gel mit guter Festigungswirkung.

Beispiel 36

Haarsprayformulierung (Standardformulierung)

6,3 Gew.-%     N-Vinylcaprolactam/tert.-Butylacrylat/2-(Dimethylamino)-ethylmethacrylat-Polymerisat
                aus Beispiel 25
33,7 Gew.-%     Ethanol
60,0 Gew.-%     Propan/n-Butan (25:75)

Der Trübungspunkt lag unterhalb von -35°C. Ein überwiegender Teil des Ethanols konnte durch n-Pentan oder n-Hexan ersetzt werden, ohne daß sich die haarfestigende Wirkung verschlechterte.

Beispiel 37

Haarsprayformulierung

4,1 Gew.-%     N-Vinylcaprolactam/tert.-Butylacrylat/Methacrylsäure-Polymerisat aus Beispiel 19, welches
                in zu 75 % mit 2-Amino-2-methylpropanol neutralisierter Form vorliegt
35,9 Gew.-%     Ethanol
60,0 Gew.-%     n-Butan

Der Trübungspunkt lag unterhalb von -35°C.

Beispiel 38

Haarsprayformulierung

8,5 Gew.-%     N-Vinylcaprolactam/tert.-Butylacrylat/Methacrylsäure-Polymerisat aus Beispiel 16, welchen
                in zu 75 % mit 2-Amino-2-methylpropanol neutralisierter Form vorliegt
51,5 Gew.-%     Ethanol
40,0 Gew.-%     n-Butan

Der Trübungspunkt lag unterhalb von -35°C.

Beispiel 39

Haarsprayformulierung

| 12,0 Gew.-% | N-Vinylcaprolactam/N-Vinylimidazol/n-Vinylpyrrolidon-Polymerisat aus Beispiel 9 |
| 38,0 Gew.-% | Ethanol |
| 50,0 Gew.-% | n-Butan |

Der Trübungspunkt lag unterhalb von -35°C.

## Patentansprüche

### Patentansprüche für folgende Vertragsstaaten : DE, FR, GB, IT, NL

1.  Haarfestigungs- und Haarpflegemittel, enthaltend neben den hierfür üblichen Bestandteilen als Filmbildner Polymerisate auf der Basis von N-Vinylcaprolactam, die aus
    Gruppe I
    - 35 bis 90 Gew.-% N-Vinylcaprolactam (Monomeres A)
    - 10 bis 65 Gew.-% N-Vinylimidazol (Monomeres B)
    - 0 bis 4 Gew.-% weiterer radikalisch copolymerisierbarer Monomerer E
    oder Gruppe II
    - 35 bis 65 Gew.-% N-Vinylcaprolactam (Monomeres A)
    - 35 bis 65 Gew.-% einer Mischung aus 5 bis 50 Gew.-Teilen N-Vinylimidazol (Monomeres B) und 10 bis 60 Gew.-Teilen N-Vinylpyrrolidon (Monomeres C)
    - 0 bis 4 Gew.% weiterer radikalisch copolymerisierbarer Monomerer E
    oder Gruppe III
    - 35 bis 85 Gew.-% N-Vinylcaprolactam (Monomeres A)
    - 15 bis 65 Gew.-% einer Mischung aus 5 bis 15 Gew.-Teilen N-Vinylimidazol (Monomeres B) und 10 bis 35 Gew.-Teilen eines Alkylacrylates oder Alkylmethacrylates mit 1 bis 4 C-Atomen im Alkylrest (Monomeres D)
    - 0 bis 4 Gew.-% weiterer radikalisch copolymerisierbarer Monomerer E
    oder Gruppe IV
    - 35 bis 75 Gew.-% N-Vinylcaprolactam (Monomeres A)
    - 25 bis 65 Gew.-% einer Mischung aus 20 bis 60 Gew.-Teilen des Monomeren D und 5 bis 15 Gew.-Teilen Acrylsäure der Methacrylsäure (Monomeres F)
    - 0 bis 4 Gew.-% weiterer radikalisch copolymerisierbarer Monomerer E
    oder Gruppe V
    - 35 bis 80 Gew.-% N-Vinylcaprolactam (Monomeres A)
    - 20 bis 65 Gew.-% einer Mischung aus 15 bis 50 Gew.-Teilen des Monomeren D und 5 bis 15 Gew.-Teilen eines Alkylacrylates oder Alkylmethacrylates mit 1 bis 4 C-Atomen im Alkylrest, wobei dieser zusätzlich eine unsubstituierte oder mit $C_1$-$C_4$-Alkylresten substituierte Aminogruppe trägt (Monomeres G)
    - 0 bis 4 Gew.-% weiterer radikalisch copolymerisierbarer Monomerer E
    aufgebaut sind, wobei vorhandene Carboxylgruppen der Polymerisate teilweise oder vollständig durch Amine neutralisiert sind.

2.  Copolymerisate, erhältlich durch radikalische Polymerisation von
    - 35 bis 95 Gew.-% N-Vinylcaprolactam (Monomeres A)
    - 5 bis 65 Gew.-% einer Mischung aus 5 bis 15 Gew.-Teilen N-Vinylimidazol (Monomeres B) und 10 bis 35 Gew.-Teilen eines Alkylacrylates oder Alkylmethacrylates mit 1 bis 4 C-Atomen im Alkylrest (Monomeres D)
    - 0 bis 4 Gew.-% weiterer radikalisch copolymerisierbarer Monomerer E.

3.  Copolymerisate, erhältlich durch radikalische Polymerisation von
    - 35 bis 95 Gew.-% N-Vinylcaprolactam (Monomeres A)
    - 5 bis 65 Gew.-% einer Mischung aus 15 bis 50 Gew.-Teilen eines Alkylacrylates oder Alkylmethacrylates mit 1 bis 4 C-Atomen im Alkylrest (Monomeres D) und 5 bis 15 Gew.-Teilen eines Alkylacrylates oder Alkylmethacrylates mit 1 bis 4 C-Atomen im Alkylrest, wobei dieser zusätzlich eine un-

substituierte oder mit $C_1$-$C_4$-Alkylresten substituierte Aminogruppe trägt (Monomeres G)
- 0 bis 4 Gew.-% weiterer radikalisch copolymerisierbarer Monomerer E.

4.  Verwendung von Polymerisaten auf der Basis von N-Vinylcaprolactam, die aus
Gruppe I
- 35 bis 90 Gew.-% N-Vinylcaprolactam (Monomeres A)
- 10 bis 65 Gew.-% N-Vinylimidazol (Monomeres B)
- 0 bis 4 Gew.-% weiterer radikalisch copolymerisierbarer Monomerer E
oder Gruppe II
- 35 bis 65 Gew.-% N-Vinylcaprolactam (Monomeres A)
- 35 bis 65 Gew.-% einer Mischung aus 5 bis 50 Gew.-Teilen N-Vinylimidazol (Monomeres B) und 10 bis 60 Gew.-Teilen N-Vinylpyrrolidon (Monomeres C)
- 0 bis 4 Gew.% weiterer radikalisch copolymerisierbarer Monomerer E
oder Gruppe III
- 35 bis 85 Gew.-% N-Vinylcaprolactam (Monomeres A)
- 15 bis 65 Gew.-% einer Mischung aus 5 bis 15 Gew.-Teilen N-Vinylimidazol (Monomeres B) und 10 bis 35 Gew.-Teilen eines Alkylacrylates oder Alkylmethacrylates mit 1 bis 4 C-Atomen im Alkylrest (Monomeres D)
- 0 bis 4 Gew.-% weiterer radikalisch copolymerisierbarer Monomerer E
oder Gruppe IV
- 35 bis 75 Gew.-% N-Vinylcaprolactam (Monomeres A)
- 25 bis 65 Gew.-% einer Mischung aus 20 bis 60 Gew.-Teilen des Monomeren D und 5 bis 15 Gew.-Teilen Acrylsäure der Methacrylsäure (Monomeres F)
- 0 bis 4 Gew.-% weiterer radikalisch copolymerisierbarer Monomerer E
oder Gruppe V
- 35 bis 80 Gew.-% N-Vinylcaprolactam (Monomeres A)
- 20 bis 65 Gew.-% einer Mischung aus 15 bis 50 Gew.-Teilen des Monomeren D und 5 bis 15 Gew.-Teilen eines Alkylacrylates oder Alkylmethacrylates mit 1 bis 4 C-Atomen im Alkylrest, wobei dieser zusätzlich eine unsubstituierte oder mit $C_1$-$C_4$-Alkylresten substituierte Aminogruppe trägt (Monomeres G)
- 0 bis 4 Gew.-% weiterer radikalisch copolymerisierbarer Monomerer E
aufgebaut sind, wobei vorhandene Carboxylgruppen der Polymerisate teilweise oder vollständig durch Amine neutralisiert sind, als Filmbildner in Haarfestigungs- und Haarpflegemitteln.

5.  Haarfestigungsmittel in Form von Sprayzubereitungen, enthaltend neben hierbei üblichen Lösungsmitteln und Treibmitteln 0,1 bis 20 Gew.-% Polymerisate auf der Basis von Vinylcaprolactam, die aus
Gruppe I
- 35 bis 90 Gew.-% N-Vinylcaprolactam (Monomeres A)
- 10 bis 65 Gew.-% N-Vinylimidazol (Monomeres B)
- 0 bis 4 Gew.-% weiterer radikalisch copolymerisierbarer Monomerer E
oder Gruppe II
- 35 bis 65 Gew.-% N-Vinylcaprolactam (Monomeres A)
- 35 bis 65 Gew.-% einer Mischung aus 5 bis 50 Gew.-Teilen N-Vinylimidazol (Monomeres B) und 10 bis 60 Gew.-Teilen N-Vinylpyrrolidon (Monomeres C)
- 0 bis 4 Gew.% weiterer radikalisch copolymerisierbarer Monomerer E oder Gruppe III
- 35 bis 85 Gew.-% N-Vinylcaprolactam (Monomeres A)
- 15 bis 65 Gew.-% einer Mischung aus 5 bis 15 Gew.-Teilen N-Vinylimidazol (Monomeres B) und 10 bis 35 Gew.-Teilen eines Alkylacrylates oder Alkylmethacrylates mit 1 bis 4 C-Atomen im Alkylrest (Monomeres D)
- 0 bis 4 Gew.-% weiterer radikalisch copolymerisierbarer Monomerer E
oder Gruppe IV
- 35 bis 75 Gew.-% N-Vinylcaprolactam (Monomeres A)
- 25 bis 65 Gew.-% einer Mischung aus 20 bis 60 Gew.-Teilen des Monomeren D und 5 bis 15 Gew.-Teilen Acrylsäure der Methacrylsäure (Monomeres F)
- 0 bis 4 Gew.-% weiterer radikalisch copolymerisierbarer Monomerer E
oder Gruppe V
- 35 bis 80 Gew.-% N-Vinylcaprolactam (Monomeres A)
- 20 bis 65 Gew.-% einer Mischung aus 15 bis 50 Gew.-Teilen des Monomeren D und 5 bis 15 Gew.-

Teilen eines Alkylacrylates oder Alkylmethacrylates mit 1 bis 4 C-Atomen im Alkylrest, wobei dieser zusätzlich eine unsubstituierte oder mit $C_1$-$C_4$-Alkylresten substituierte Aminogruppe trägt (Monomeres G)

- 0 bis 4 Gew.-% weiterer radikalisch copolymerisierbarer Monomerer E

aufgebaut sind, wobei vorhandene Carboxylgruppen der Polymerisate teilweise oder vollständig durch Amine neutralisiert sind.

**Patentansprüche für folgenden Vertragsstaat : ES**

1. Verfahren zur Herstellung von Haarfestigungs- und Haarpflegemitteln, dadurch gekennzeichnet, daß man zu den hierfür üblichen Bestandteilen als Filmbildner Polymerisate auf der Basis von N-Vinylcaprolactam, die aus
   Gruppe I
   - 35 bis 90 Gew.-% N-Vinylcaprolactam (Monomeres A)
   - 10 bis 65 Gew.-% N-Vinylimidazol (Monomeres B)
   - 0 bis 4 Gew.-% weiterer radikalisch copolymerisierbarer Monomerer E
   oder Gruppe II
   - 35 bis 65 Gew.-% N-Vinylcaprolactam (Monomeres A)
   - 35 bis 65 Gew.-% einer Mischung aus 5 bis 50 Gew.-Teilen N-Vinylimidazol (Monomeres B) und 10 bis 60 Gew.-Teilen N-Vinylpyrrolidon (Monomeres C)
   - 0 bis 4 Gew.% weiterer radikalisch copolymerisierbarer Monomerer E
   oder Gruppe III
   - 35 bis 85 Gew.-% N-Vinylcaprolactam (Monomeres A)
   - 15 bis 65 Gew.-% einer Mischung aus 5 bis 15 Gew.-Teilen N-Vinylimidazol (Monomeres B) und 10 bis 35 Gew.-Teilen eines Alkylacrylates oder Alkylmethacrylates mit 1 bis 4 C-Atomen im Alkylrest (Monomeres D)
   - 0 bis 4 Gew.-% weiterer radikalisch copolymerisierbarer Monomerer E
   oder Gruppe IV
   - 35 bis 75 Gew.-% N-Vinylcaprolactam (Monomeres A)
   - 25 bis 65 Gew.-% einer Mischung aus 20 bis 60 Gew.-Teilen des Monomeren D und 5 bis 15 Gew.-Teilen Acrylsäure der Methacrylsäure (Monomeres F)
   - 0 bis 4 Gew.-% weiterer radikalisch copolymerisierbarer Monomerer E
   oder Gruppe V
   - 35 bis 80 Gew.-% N-Vinylcaprolactam (Monomeres A)
   - 20 bis 65 Gew.-% einer Mischung aus 15 bis 50 Gew.-Teilen des Monomeren D und 5 bis 15 Gew.-Teilen eines Alkylacrylates oder Alkylmethacrylates mit 1 bis 4 C-Atomen im Alkylrest, wobei dieser zusätzlich eine unsubstituierte oder mit $C_1$-$C_4$-Alkylresten substituierte Aminogruppe trägt (Monomeres G)
   - 0 bis 4 Gew.-% weiterer radikalisch copolymerisierbarer Monomerer E
   aufgebaut sind, wobei vorhandene Carboxylgruppen der Polymerisate teilweise oder vollständig durch Amine neutralisiert sind, zufügt.

2. Verfahren zur Herstellung von Copolymerisaten, dadurch gekennzeichnet, daß man
   - 35 bis 95 Gew.-% N-Vinylcaprolactam (Monomeres A)
   - 5 bis 65 Gew.-% einer Mischung aus 5 bis 15 Gew.-Teilen N-Vinylimidazol (Monomeres B) und 10 bis 35 Gew.-Teilen eines Alkylacrylates oder Alkylmethacrylates mit 1 bis 4 C-Atomen im Alkylrest (Monomeres D)
   - 0 bis 4 Gew.-% weiterer radikalisch copolymerisierbarer Monomerer E
   miteinander radikalisch polymerisiert.

3. Verfahren zur Herstellung von Copolymerisaten, dadurch gekennzeichnet, daß man
   - 35 bis 95 Gew.-% N-Vinylcaprolactam (Monomeres A)
   - 5 bis 65 Gew.-% einer Mischung aus 15 bis 50 Gew.-Teilen eines Alkylacrylates oder Alkylmethacrylates mit 1 bis 4 C-Atomen im Alkylrest (Monomeres D) und 5 bis 15 Gew.-Teilen eines Alkylacrylates oder Alkylmethacrylates mit 1 bis 4 C-Atomen im Alkylrest, wobei dieser zusätzlich eine unsubstituierte oder mit $C_1$-$C_4$-Alkylresten substituierte Aminogruppe trägt (Monomeres G)
   - 0 bis 4 Gew.-% weiterer radikalisch copolymerisierbarer Monomerer E
   miteinander radikalisch polymerisiert.

**4.** Verfahren zur Herstellung von Haarfestigungsmitteln in Form von Sprayzubereitungen, dadurch gekennzeichnet, daß man hierbei übliche Lösungsmittel und Treibmittel mit 0,1 bis 20 Gew.-% Polymerisaten auf der Basis von Vinylcaprolactam, die aus

Gruppe I
- 35 bis 90 Gew.-% N-Vinylcaprolactam (Monomeres A)
- 10 bis 65 Gew.-% N-Vinylimidazol (Monomeres B)
- 0 bis 4 Gew.-% weiterer radikalisch copolymerisierbarer Monomerer E

oder Gruppe II
- 35 bis 65 Gew.-% N-Vinylcaprolactam (Monomeres A)
- 35 bis 65 Gew.-% einer Mischung aus 5 bis 50 Gew.-Teilen N-Vinylimidazol (Monomeres B) und 10 bis 60 Gew.-Teilen N-Vinylpyrrolidon (Monomeres C)
- 0 bis 4 Gew.% weiterer radikalisch copolymerisierbarer Monomerer E

oder Gruppe III
- 35 bis 85 Gew.-% N-Vinylcaprolactam (Monomeres A)
- 15 bis 65 Gew.-% einer Mischung aus 5 bis 15 Gew.-Teilen N-Vinylimidazol (Monomeres B) und 10 bis 35 Gew.-Teilen eines Alkylacrylates oder Alkylmethacrylates mit 1 bis 4 C-Atomen im Alkylrest (Monomeres D)
- 0 bis 4 Gew.-% weiterer radikalisch copolymerisierbarer Monomerer E

oder Gruppe IV
- 35 bis 75 Gew.-% N-Vinylcaprolactam (Monomeres A)
- 25 bis 65 Gew.-% einer Mischung aus 20 bis 60 Gew.-Teilen des Monomeren D und 5 bis 15 Gew.-Teilen Acrylsäure der Methacrylsäure (Monomeres F)
- 0 bis 4 Gew.-% weiterer radikalisch copolymerisierbarer Monomerer E

oder Gruppe V
- 35 bis 80 Gew.-% N-Vinylcaprolactam (Monomeres A)
- 20 bis 65 Gew.-% einer Mischung aus 15 bis 50 Gew.-Teilen des Monomeren D und 5 bis 15 Gew.-Teilen eines Alkylacrylates oder Alkylmethacrylates mit 1 bis 4 C-Atomen im Alkylrest, wobei dieser zusätzlich eine unsubstituierte oder mit $C_1$-$C_4$-Alkylresten substituierte Aminogruppe trägt (Monomeres G)
- 0 bis 4 Gew.-% weiterer radikalisch copolymerisierbarer Monomerer E

aufgebaut sind, wobei vorhandene Carboxylgruppen der Polymerisate teilweise oder vollständig durch Amine neutralisiert sind, mischt.

## Claims

## Claims for the following Contracting States : DE, FR, GB, IT, NL

**1.** Hair setting and hair care compositions containing, as well as the customary ingredients for this purpose, film formers comprising polymers based on N-vinylcaprolactam formed from

group I
- from 35 to 90% by weight of N-vinylcaprolactam (monomer A)
- from 10 to 65% by weight of N-vinylimidazole (monomer B)
- from 0 to 4% by weight of further free-radically copolymerizable monomers E

or group II
- from 35 to 65% by weight of N-vinylcaprolactam (monomer A)
- from 35 to 65% by weight of a mixture of from 5 to 50 parts by weight of N-vinylimidazole (monomer B) and from 10 to 60 parts by weight of N-vinylpyrrolidone (monomer C)
- from 0 to 4% by weight of further free-radically copolymerizable monomers E

or group III
- from 35 to 85% by weight of N-vinylcaprolactam (monomer A)
- from 15 to 65% by weight of a mixture of from 5 to 15 parts by weight of N-vinylimidazole (monomer B) and from 10 to 35 parts by weight of an alkyl acrylate or methacrylate having from 1 to 4 carbon atoms in the alkyl moiety (monomer D)
- from 0 to 4% by weight of further free-radically copolymerizable monomers E or group IV
- from 35 to 75% by weight of N-vinylcaprolactam (monomer A)
- from 25 to 65% by weight of a mixture of from 20 to 60 parts by weight of monomer D and from 5 to 15 parts by weight of acrylic or methacrylic acid (monomer F)

- from 0 to 4% by weight of further free-radically copolymerizable monomers E

or group V

- from 35 to 80% by weight of N-vinylcaprolactam (monomer A)
- from 20 to 65% by weight of a mixture of from 15 to 50 parts by weight of monomer D and from 5 to 15 parts by weight of an alkyl acrylate or methacrylate having from 1 to 4 carbon atoms in the alkyl moiety which additionally carries an unsubstituted or $C_1$-$C_4$-alkyl-substituted amino group (monomer G)
- from 0 to 4% by weight of further free-radically copolymerizable monomers E,

any carboxyl groups of the polymers having been partly or wholly neutralized with amines.

2. Copolymers obtainable by free-radical polymerization of
- from 35 to 95% by weight of N-vinylcaprolactam (monomer A)
- from 5 to 65% by weight of a mixture of from 5 to 15 parts by weight of N-vinylimidazole (monomer B) and from 10 to 35 parts by weight of an alkyl acrylate or methacrylate having from 1 to 4 carbon atoms in the alkyl moiety (monomer D)
- from 0 to 4% by weight of further free-radically copolymerizable monomers E.

3. Copolymers obtainable by free-radical polymerization of
- from 35 to 95% by weight of N-vinylcaprolactam (monomer A)
- from 5 to 65% by weight of a mixture of from 15 to 50 parts by weight of an alkyl acrylate or methacrylate having from 1 to 4 carbon atoms in the an alkyl moiety (monomer D) and from 5 to 15 parts by weight of an alkyl acrylate or methacrylate having from 1 to 4 carbon atoms in the alkyl moiety which additionally carries an unsubstituted or $C_1$-$C_4$-alkyl-substituted amino group (monomer G)
- from 0 to 4% by weight of further free-radically copolymerizable monomers E.

4. The use as film formers in hair setting and hair care compositions of polymers based on N-vinylcaprolactam formed from

group I

- from 35 to 90% by weight of N-vinylcaprolactam (monomer A)
- from 10 to 65% by weight of N-vinylimidazole (monomer B)
- from 0 to 4% by weight of further free-radically copolymerizable monomers E

or group II

- from 35 to 65% by weight of N-vinylcaprolactam (monomer A)
- from 35 to 65% by weight of a mixture of from 5 to 50 parts by weight of N-vinylimidazole (monomer B) and from 10 to 60 parts by weight of N-vinylpyrrolidone (monomer C)
- from 0 to 4% by weight of further free-radically copolymerizable monomers E

or group III

- from 35 to 85% by weight of N-vinylcaprolactam (monomer A)
- from 15 to 65% by weight of a mixture of from 5 to 15 parts by weight of N-vinylimidazole (monomer B) and from 10 to 35 parts by weight of an alkyl acrylate or methacrylate having from 1 to 4 carbon atoms in the alkyl moiety (monomer D)
- from 0 to 4% by weight of further free-radically copolymerizable monomers E

or group IV

- from 35 to 75% by weight of N-vinylcaprolactam (monomer A)
- from 25 to 65% by weight of a mixture of from 20 to 60 parts by weight of monomer D and from 5 to 15 parts by weight of acrylic or methacrylic acid (monomer F)
- from 0 to 4% by weight of further free-radically copolymerizable monomers E

or group V

- from 35 to 80% by weight of N-vinylcaprolactam (monomer A)
- from 20 to 65% by weight of a mixture of from 15 to 50 parts by weight of monomer D and from 5 to 15 parts by weight of an alkyl acrylate or methacrylate having from 1 to 4 carbon atoms in the alkyl moiety which additionally carries an unsubstituted or $C_1$-$C_4$-alkyl-substituted amino group (monomer G)
- from 0 to 4% by weight of further free-radically copolymerizable monomers E,

any carboxyl groups of the polymers having been partly or wholly neutralized with amines.

5. Hair setting compositions in the form of sprays containing as well as customary solvents and propellants from 0.1 to 20% by weight of polymers based on vinylcaprolactam formed from

group I
- from 35 to 90% by weight of N-vinylcaprolactam (monomer A)
- from 10 to 65% by weight of N-vinylimidazole (monomer B)
- from 0 to 4% by weight of further free-radically copolymerizable monomers E

or group II
- from 35 to 65% by weight of N-vinylcaprolactam (monomer A)
- from 35 to 65% by weight of a mixture of from 5 to 50 parts by weight of N-vinylimidazole (monomer B) and from 10 to 60 parts by weight of N-vinylpyrrolidone (monomer C)
- from 0 to 4% by weight of further free-radically copolymerizable monomers E

or group III
- from 35 to 85% by weight of N-vinylcaprolactam (monomer A)
- from 15 to 65% by weight of a mixture of from 5 to 15 parts by weight of N-vinylimidazole (monomer B) and from 10 to 35 parts by weight of an alkyl acrylate or methacrylate having from 1 to 4 carbon atoms in the alkyl moiety (monomer D)
- from 0 to 4% by weight of further free-radically copolymerizable monomers E

or group IV
- from 35 to 75% by weight of N-vinylcaprolactam (monomer A)
- from 25 to 65% by weight of a mixture of from 20 to 60 parts by weight of monomer D and from 5 to 15 parts by weight of acrylic or methacrylic acid (monomer F)
- from 0 to 4% by weight of further free-radically copolymerizable monomers E

or group V
- from 35 to 80% by weight of N-vinylcaprolactam (monomer A)
- from 20 to 65% by weight of a mixture of from 15 to 50 parts by weight of monomer D and from 5 to 15 parts by weight of an alkyl acrylate or methacrylate having from 1 to 4 carbon atoms in the alkyl moiety which additionally carries an unsubstituted or $C_1$-$C_4$-alkyl-substituted amino group (monomer G)
- from 0 to 4% by weight of further free-radically copolymerizable monomers E,

any carboxyl groups of the polymers having been partly or wholly neutralized with amines.

**Claims for the following Contracting State : ES**

1. A process for preparing hair setting and hair care compositions, which comprises adding to the customary ingredients for this purpose film formers comprising polymers based on N-vinylcaprolactam formed from

group I
- from 35 to 90% by weight of N-vinylcaprolactam (monomer A)
- from 10 to 65% by weight of N-vinylimidazole (monomer B)
- from 0 to 4% by weight of further free-radically copolymerizable monomers E

or group II
- from 35 to 65% by weight of N-vinylcaprolactam (monomer A)
- from 35 to 65% by weight of a mixture of from 5 to 50 parts by weight of N-vinylimidazole (monomer B) and from 10 to 60 parts by weight of N-vinylpyrrolidone (monomer C)
- from 0 to 4% by weight of further free-radically copolymerizable monomers E

or group III
- from 35 to 85% by weight of N-vinylcaprolactam (monomer A)
- from 15 to 65% by weight of a mixture of from 5 to 15 parts by weight of N-vinylimidazole (monomer B) and from 10 to 35 parts by weight of an alkyl acrylate or methacrylate having from 1 to 4 carbon atoms in the alkyl moiety (monomer D)
- from 0 to 4% by weight of further free-radically copolymerizable monomers E

or group IV
- from 35 to 75% by weight of N-vinylcaprolactam (monomer A)
- from 25 to 65% by weight of a mixture of from 20 to 60 parts by weight of monomer D and from 5 to 15 parts by weight of acrylic or methacrylic acid (monomer F)
- from 0 to 4% by weight of further free-radically copolymerizable monomers E

or group V
- from 35 to 80% by weight of N-vinylcaprolactam (monomer A)
- from 20 to 65% by weight of a mixture of from 15 to 50 parts by weight of monomer D and from 5 to 15 parts by weight of an alkyl acrylate or methacrylate having from 1 to 4 carbon atoms in the alkyl moiety which additionally carries an unsubstituted or $C_1$-$C_4$-alkyl-substituted amino group (monomer G)
- from 0 to 4% by weight of further free-radically copolymerizable monomers E,

any carboxyl groups of the polymers having been partly or wholly neutralized with amines.

2. A process for preparing copolymers, which comprises free-radically copolymerizing
   - from 35 to 95% by weight of N-vinylcaprolactam (monomer A)
   - from 5 to 65% by weight of a mixture of from 5 to 15 parts by weight of N-vinylimidazole (monomer B) and from 10 to 35 parts by weight of an alkyl acrylate or methacrylate having from 1 to 4 carbon atoms in the alkyl moiety (monomer D)
   - from 0 to 4% by weight of further free-radically copolymerizable monomers E.

3. A process for preparing copolymers, which comprises free-radically copolymerizing
   - from 35 to 95% by weight of N-vinylcaprolactam (monomer A)
   - from 5 to 65% by weight of a mixture of from 15 to 50 parts by weight of an alkyl acrylate or methacrylate having from 1 to 4 carbon atoms in the alkyl moiety (monomer D) and from 5 to 15 parts by weight of an alkyl acrylate or methacrylate having from 1 to 4 carbon atoms in the alkyl moiety which additionally carries an unsubstituted or $C_1$-$C_4$-alkyl-substituted amino group (monomer G)
   - from 0 to 4% by weight of further free-radically copolymerizable monomers E.

4. A process for preparing hair setting compositions in the form of sprays, which comprises mixing customary solvents and propellants with from 0.1 to 20% by weight of polymers based on vinylcaprolactam formed from group I
   - from 35 to 90% by weight of N-vinylcaprolactam (monomer A)
   - from 10 to 65% by weight of N-vinylimidazole (monomer B)
   - from 0 to 4% by weight of further free-radically copolymerizable monomers E

   or group II
   - from 35 to 65% by weight of N-vinylcaprolactam (monomer A)
   - from 35 to 65% by weight of a mixture of from 5 to 50 parts by weight of N-vinylimidazole (monomer B) and from 10 to 60 parts by weight of N-vinylpyrrolidone (monomer C)
   - from 0 to 4% by weight of further free-radically copolymerizable monomers E

   or group III
   - from 35 to 85% by weight of N-vinylcaprolactam (monomer A)
   - from 15 to 65% by weight of a mixture of from 5 to 15 parts by weight of N-vinylimidazole (monomer B) and from 10 to 35 parts by weight of an alkyl acrylate or methacrylate having from 1 to 4 carbon atoms in the alkyl moiety (monomer D)
   - from 0 to 4% by weight of further free-radically copolymerizable monomers E

   or group IV
   - from 35 to 75% by weight of N-vinylcaprolactam (monomer A)
   - from 25 to 65% by weight of a mixture of from 20 to 60 parts by weight of monomer D and from 5 to 15 parts by weight of acrylic or methacrylic acid (monomer F)
   - from 0 to 4% by weight of further free-radically copolymerizable monomers E

   or group V
   - from 35 to 80% by weight of N-vinylcaprolactam (monomer A)
   - from 20 to 65% by weight of a mixture of from 15 to 50 parts by weight of monomer D and from 5 to 15 parts by weight of an alkyl acrylate or methacrylate having from 1 to 4 carbon atoms in the alkyl moiety which additionally carries an unsubstituted or $C_1$-$C_4$-alkyl-substituted amino group (monomer G)
   - from 0 to 4% by weight of further free-radically copolymerizable monomers E,

   any carboxyl groups of the polymers having been partly or wholly neutralized with amines.

## Revendications

## Revendications pour les Etats contractants suivants : DE, FR, GB, IT, NL

1. Produit fixateur et de soin des cheveux, contenant comme filmogène, en dehors des constituants utilisés ordinairement dans de tels produits, des polymères à base de N-vinylcaprolactame qui sont constitués par

Groupe I

- 35 à 90% en poids de N-vinylcaprolactame (monomère A)
- 10 à 65% en poids de N-vinylimidazole (monomère B)
- 0 à 4% en poids d'autres monomères E susceptibles de copolymérisation radicalaire, ou

Groupe II

- 35 à 65% en poids de N-vinylcaprolactame (monomère A)
- 35 à 65% en poids d'un mélange de 5 à 50 parties en poids de N-vinylimidazole (monomère B) et de 10 à 60 parties en poids de N-vinylpyrrolidone (monomère C)
- 0 à 4% en poids d'autres monomères E susceptibles de copolymérisation radicalaire, ou

Groupe III

- 35 à 85% en poids de N-vinylcaprolactame (monomère A)
- 15 à 65% en poids d'un mélange de 5 à 15 parties en poids de N-vinylimidazole (monomère B) et de 10 à 35 parties en poids d'un acrylate d'alkyle ou d'un méthacrylate d'alkyle à 1-4 atomes de carbone dans le reste alkyle (monomère D)
- 0 à 4% en poids d'autres monomères E susceptibles de copolymérisation radicalaire, ou

Groupe IV

- 35 à 75% en poids de N-vinylcaprolactame (monomère A)
- 25 à 65% en poids d'un mélange de 20 à 60 parties en poids du monomère D et de 5 à 15 parties en poids d'acide acrylique ou d'acide méthacrylique (monomère F)
- 0 à 4% en poids d'autres monomères E susceptibles de copolymérisation radicalaire, ou

Groupe V

- 35 à 80% en poids de N-vinylcaprolactame (monomère A)
- 20 à 65% en poids d'un mélange de 15 à 50 parties en poids du monomère D et de 5 à 15 parties en poids d'un acrylate d'alkyle ou d'un méthacrylate d'alkyle à 1-4 atomes de carbone dans le reste alkyle, ce dernier portant en plus un groupement amino non substitué ou substitué par des restes alkyle en $C_1$-$C_4$ (monomère G),
- 0 à 4% en poids d'autres monomères E susceptibles de copolymérisation radicalaire,

les groupements carboxyle présents dans les polymères étant partiellement ou complètement neutralisés par des amines.

2. Copolymères, obtenus par polymérisation radicalaire de

- 35 à 95% en poids de N-vinylcaprolactame (monomère A)
- 5 à 65% en poids d'un mélange de 5 à 15 parties en poids de N-vinylimidazole (monomère B) et de 10 à 35 parties en poids d'un acrylate d'alkyle ou d'un méthacrylate d'alkyle à 1-4 atomes de carbone dans le reste alkyle (monomère D)
- 0 à 4% en poids d'autres monomères E susceptibles de copolymérisation radicalaire.

3. Copolymères, obtenus par polymérisation radicalaire de

- 35 à 95% en poids de N-vinylcaprolactame (monomère A)
- 5 à 65% en poids d'un mélange de 15 à 50 parties en poids d'un acrylate d'alkyle ou d'un méthacrylate d'alkyle à 1-4 atomes de carbone dans le reste alkyle (monomère D) et de 5 à 15 parties en poids d'un acrylate d'alkyle ou d'un méthacrylate d'alkyle à 1-4 atomes de carbone dans le reste alkyle, ce dernier portant en plus un groupement amino non substitué ou substitué par des restes alkyle en $C_1$-$C_4$ (monomère G),
- 0 à 4% en poids d'autres monomères E susceptibles de copolymérisation radicalaire.

4. Utilisation, comme filmogène dans des produits fixateurs et de soin des cheveux, de polymères à base de N-vinylcaprolactame qui sont constitués par

Groupe I

- 35 à 90% en poids de N-vinylcaprolactame (monomère A)
- 10 à 65% en poids de N-vinylimidazole (monomère B)
- 0 à 4% en poids d'autres monomères E susceptibles de copolymérisation radicalaire, ou

Groupe II

- 35 à 65% en poids de N-vinylcaprolactame (monomère A)
- 35 à 65% en poids d'un mélange de 5 à 50 parties en poids de N-vinylimidazole (monomère B) et de 10 à 60 parties en poids de N-vinylpyrrolidone (monomère C)
- 0 à 4% en poids d'autres monomères E susceptibles de copolymérisation radicalaire, ou

Groupe III

- 35 à 85% en poids de N-vinylcaprolactame (monomère A)
- 15 à 65% en poids d'un mélange de 5 à 15 parties en poids de N-vinylimidazole (monomère B) et de 10 à 35 parties en poids d'un acrylate d'alkyle ou d'un méthacrylate d'alkyle à 1-4 atomes de carbone dans le reste alkyle (monomère D)
- 0 à 4% en poids d'autres monomères E susceptibles de copolymérisation radicalaire, ou

Groupe IV
- 35 à 75% en poids de N-vinylcaprolactame (monomère A)
- 25 à 65% en poids d'un mélange de 20 à 60 parties en poids du monomère D et de 5 à 15 parties en poids d'acide acrylique ou d'acide méthacrylique (monomère F)
- 0 à 4% en poids d'autres monomères E susceptibles de copolymérisation radicalaire, ou

Groupe V
- 35 à 80% en poids de N-vinylcaprolactame (monomère A)
- 20 à 65% en poids d'un mélange de 15 à 50 parties en poids du monomère D et de 5 à 15 parties en poids d'un acrylate d'alkyle ou d'un méthacrylate d'alkyle à 1-4 atomes de carbone dans le reste alkyle, ce dernier portant en plus un groupement amino non substitué ou substitué par des restes alkyle en $C_1$-$C_4$ (monomère G),
- 0 à 4% en poids d'autres monomères E susceptibles de copolymérisation radicalaire,

les groupements carboxyle présents dans les polymères étant partiellement ou complètement neutralisés par des amines.

5. Produit fixateur capillaire sous forme de préparation à pulvériser, contenant comme filmogène, en dehors des constituants et agents propulseurs utilisés ordinairement dans de tels produits, de 0,1 à 20% en poids de polymères à base de N-vinylcaprolactame qui sont constitués par

Groupe I
- 35 à 90% en poids de N-vinylcaprolactame (monomère A)
- 10 à 65% en poids de N-vinylimidazole (monomère B)
- 0 à 4% en poids d'autres monomères E susceptibles de copolymérisation radicalaire, ou

Groupe II
- 35 à 65% en poids de N-vinylcaprolactame (monomère A)
- 35 à 65% en poids d'un mélange de 5 à 50 parties en poids de N-vinylimidazole (monomère B) et de 10 à 60 parties en poids de N-vinylpyrrolidone (monomère C)
- 0 à 4% en poids d'autres monomères E susceptibles de copolymérisation radicalaire, ou

Groupe III
- 35 à 85% en poids de N-vinylcaprolactame (monomère A)
- 15 à 65% en poids d'un mélange de 5 à 15 parties en poids de N-vinylimidazole (monomère B) et de 10 à 35 parties en poids d'un acrylate d'alkyle ou d'un méthacrylate d'alkyle à 1-4 atomes de carbone dans le reste alkyle (monomère D)
- 0 à 4% en poids d'autres monomères E susceptibles de copolymérisation radicalaire, ou

Groupe IV
- 35 à 75% en poids de N-vinylcaprolactame (monomère A)
- 25 à 65% en poids d'un mélange de 20 à 60 parties en poids du monomère D et de 5 à 15 parties en poids d'acide acrylique ou d'acide méthacrylique (monomère F)
- 0 à 4% en poids d'autres monomères E susceptibles de copolymérisation radicalaire, ou

Groupe V
- 35 à 80% en poids de N-vinylcaprolactame (monomère A)
- 20 à 65% en poids d'un mélange de 15 à 50 parties en poids du monomère D et de 5 à 15 parties en poids d'un acrylate d'alkyle ou d'un méthacrylate d'alkyle à 1-4 atomes de carbone dans le reste alkyle, ce dernier portant en plus un groupement amino non substitué ou substitué par des restes alkyle en $C_1$-$C_4$ (monomère G),
- 0 à 4% en poids d'autres monomères E susceptibles de copolymérisation radicalaire,

les groupements carboxyle présents dans les polymères étant partiellement ou complètement neutralisés par des amines.

## Revendications pour l'Etat contractant suivant : ES

1. Procédé de préparation de produits fixateurs et de soin des cheveux, caractérisé en ce qu'on ajoute comme filmogène, aux constituants utilisés ordinairement dans de tels produits, des polymères à base de N-vinylcaprolactame qui sont constitués par

Groupe I
- 35 à 90% en poids de N-vinylcaprolactame (monomère A)
- 10 à 65% en poids de N-vinylimidazole (monomère B)
- 0 à 4% en poids d'autres monomères E susceptibles de copolymérisation radicalaire, ou

Groupe II
- 35 à 65% en poids de N-vinylcaprolactame (monomère A)
- 35 à 65% en poids d'un mélange de 5 à 50 parties en poids de N-vinylimidazole (monomère B) et de 10 à 60 parties en poids de N-vinylpyrrolidone (monomère C)
- 0 à 4% en poids d'autres monomères E susceptibles de copolymérisation radicalaire, ou

Groupe III
- 35 à 85% en poids de N-vinylcaprolactame (monomère A)
- 15 à 65% en poids d'un mélange de 5 à 15 parties en poids de N-vinylimidazole (monomère B) et de 10 à 35 parties en poids d'un acrylate d'alkyle ou d'un méthacrylate d'alkyle à 1-4 atomes de carbone dans le reste alkyle (monomère D)
- 0 à 4% en poids d'autres monomères E susceptibles de copolymérisation radicalaire, ou

Groupe IV
- 35 à 75% en poids de N-vinylcaprolactame (monomère A)
- 25 à 65% en poids d'un mélange de 20 à 60 parties en poids du monomère D et de 5 à 15 parties en poids d'acide acrylique ou d'acide méthacrylique (monomère F)
- 0 à 4% en poids d'autres monomères E susceptibles de copolymérisation radicalaire, ou

Groupe V
- 35 à 80% en poids de N-vinylcaprolactame (monomère A)
- 20 à 65% en poids d'un mélange de 15 à 50 parties en poids du monomère D et de 5 à 15 parties en poids d'un acrylate d'alkyle ou d'un méthacrylate d'alkyle à 1-4 atomes de carbone dans le reste alkyle, ce dernier portant en plus un groupement amino non substitué ou substitué par des restes alkyle en $C_1$-$C_4$ (monomère G),
- 0 à 4% en poids d'autres monomères E susceptibles de copolymérisation radicalaire,

les groupements carboxyle présents dans les polymères étant partiellement ou complètement neutralisés par des amines.

2. Procédé de préparation de copolymères, caractérisé en ce qu'on polymérise ensemble par réaction radicalaire
- 35 à 95% en poids de N-vinylcaprolactame (monomère A)
- 5 à 65% en poids d'un mélange de 5 à 15 parties en poids de N-vinylimidazole (monomère B) et de 10 à 35 parties en poids d'un acrylate d'alkyle ou d'un méthacrylate d'alkyle à 1-4 atomes de carbone dans le reste alkyle (monomère D)
- 0 à 4% en poids d'autres monomères E susceptibles de copolymérisation radicalaire.

3. Procédé de préparation de copolymères, caractérisé en ce qu'on polymérise ensemble par réaction radicalaire
- 35 à 95% en poids de N-vinylcaprolactame (monomère A)
- 5 à 65% en poids d'un mélange de 15 à 50 parties en poids d'un acrylate d'alkyle ou d'un méthacrylate d'alkyle à 1-4 atomes de carbone dans le reste alkyle (monomère D) et de 5 à 15 parties en poids d'un acrylate d'alkyle ou d'un méthacrylate d'alkyle à 1-4 atomes de carbone dans le reste alkyle, ce dernier portant en plus un groupement amino non substitué ou substitué par des restes alkyle en $C_1$-$C_4$ (monomère G),
- 0 à 4% en poids d'autres monomères E susceptibles de copolymérisation radicalaire.

4. Procédé de préparation de produits fixateurs capillaires sous forme de préparations à pulvériser, caractérisé en ce qu'on mélange des solvants et agents propulseurs utilisés ordinairement dans de tels produits avec 0,1 à 20% en poids de polymères à base de N-vinylcaprolactame qui sont constitués par

Groupe I
- 35 à 90% en poids de N-vinylcaprolactame (monomère A)
- 10 à 65% en poids de N-vinylimidazole (monomère B)
- 0 à 4% en poids d'autres monomères E susceptibles de copolymérisation radicalaire, ou

Groupe II
- 35 à 65% en poids de N-vinylcaprolactame (monomère A)
- 35 à 65% en poids d'un mélange de 5 à 50 parties en poids de N-vinylimidazole (monomère B) et de 10 à 60 parties en poids de N-vinylpyrrolidone (monomère C)
- 0 à 4% en poids d'autres monomères E susceptibles de copolymérisation radicalaire, ou
Groupe III
- 35 à 85% en poids de N-vinylcaprolactame (monomère A)
- 15 à 65% en poids d'un mélange de 5 à 15 parties en poids de N-vinylimidazole (monomère B) et de 10 à 35 parties en poids d'un acrylate d'alkyle ou d'un méthacrylate d'alkyle à 1-4 atomes de carbone dans le reste alkyle (monomère D)
- 0 à 4% en poids d'autres monomères E susceptibles de copolymérisation radicalaire, ou
Groupe IV
- 35 à 75% en poids de N-vinylcaprolactame (monomère A)
- 25 à 65% en poids d'un mélange de 20 à 60 parties en poids du monomère D et de 5 à 15 parties en poids d'acide acrylique ou d'acide méthacrylique (monomère F)
- 0 à 4% en poids d'autres monomères E susceptibles de copolymérisation radicalaire, ou
Groupe V
- 35 à 80% en poids de N-vinylcaprolactame (monomère A)
- 20 à 65% en poids d'un mélange de 15 à 50 parties en poids du monomère D et de 5 à 15 parties en poids d'un acrylate d'alkyle ou d'un méthacrylate d'alkyle à 1-4 atomes de carbone dans le reste alkyle, ce dernier portant en plus un groupement amino non substitué ou substitué par des restes alkyle en $C_1$-$C_4$ (monomère G),
- 0 à 4% en poids d'autres monomères E susceptibles de copolymérisation radicalaire, ou
les groupements carboxyle présents dans les polymères étant partiellement ou complètement neutralisés par des amines.